**Europäisches Patentamt**

**European Patent Office**

**Office européen des brevets**

(11) Veröffentlichungsnummer: **0 212 568 B1**

(12)
# EUROPÄISCHE PATENTSCHRIFT

(45) Veröffentlichungstag der Patentschrift: **06.11.91**

(51) Int. Cl.⁵: **A61K 49/00**, A61B 8/00

(21) Anmeldenummer: **86111220.9**

(22) Anmeldetag: **13.08.86**

Die Akte enthält technische Angaben, die nach dem Eingang der Anmeldung eingereicht wurden und die nicht in dieser Patentschrift enthalten sind.

(54) **Kontrastmittel für Ultraschalluntersuchungen und Verfahren zu seiner Herstellung.**

(30) Priorität: **14.08.85 DE 3529195**

(43) Veröffentlichungstag der Anmeldung:
**04.03.87 Patentblatt 87/10**

(45) Bekanntmachung des Hinweises auf die Patenterteilung:
**06.11.91 Patentblatt 91/45**

(84) Benannte Vertragsstaaten:
**AT BE CH DE FR GB IT LI LU NL SE**

(56) Entgegenhaltungen:
**EP-A- 0 123 235**
**WO-A-80/02365**
**WO-A-84/02838**

**ULTRASONIC IMAGING, 1980, Seiten 67-77, Academic Press, Inc., US; J. OPHIR et al.: "Ultrasonic backscatter from contrast producing collagen microspheres"**

(73) Patentinhaber: **Byk Gulden Lomberg Chemische Fabrik GmbH**
**Byk-Gulden-Strasse 2**
**W-7750 Konstanz(DE)**

(72) Erfinder: **Berwing, Klaus, Dr.**
**In den Nussgarten 40**
**W-6350 Bad Nauheim(DE)**
Erfinder: **Schlepper, Martin, Prof. Dr.**
**Grüner Weg 27**
**W-6350 Bad Nauheim(DE)**

**Beschreibung**

Gegenstand der Erfindung ist ein Kontrastmittel für Ultraschalluntersuchungen, insbesondere für die Echokardiographie auf der Grundlage einer Suspension von Gasbläschen in einer pharmakologisch annehmbaren, wäßrigen Trägerflüssigkeit mit einem physiologischen Elektrolytgehalt.

Die Untersuchung innerer Organe von Menschen und Tieren mit Ultraschall ist eine seit langer Zeit gut eingeführte und praktizierte diagnostische Methode, die darauf basiert, daß Ultraschallwellen im Megahertz-Bereich (oberhalb 2 MHz) an den Grenzflächen unterschiedlicher Gewebsarten reflektiert werden. Die hierdurch entstehenden Echos werden verstärkt und sichtbar gemacht. Von besonderer Bedeutung ist dabei die Kontrastmittel-Echokardiographie, die zur Diagnostik von Herzerkrankungen sowohl in der M-Mode als auch in der zweidimensionalen Echokardiographie dient. Hierbei wird durch intravenöse Injektion eines Kontrastmittels ein Ultraschallecho in jenen Bereichen hervorgerufen, in denen sich das Kontrastmittel befindet, wodurch es möglich wird, Rechtsherzerkrankungen einschließlich Vorhof- und Ventrikelseptum-Defekte zu diagnostizieren, wobei die Diagnostik von Linksherzerkrankungen nach dieser Methode daran leidet, daß es erforderlich ist, das Kontrastmittel über einen in die linke Herzkammer oder im Pulmonalkapillarbereich eingeführten Katheter zuzuführen (R.S. Meltzer et al., Brit. Heart J. 44 (1980), 390; R.S. Meltzer et al., Med. & Biol. 6 (1980), 263; C. Mortera et al., Eur. J. Cardiol. 9/6 (1979), 437; A. Reale et al., Eur. Heart J. 1 (1980), 101 und J. Roelandt et al., Echocardiology, Martinus Nijhoff, Den Haag, Boston, London (1981) 219).

Es sind bereits verschiedene Kontrastmittel für die Ultraschall-Echokardiographie beschrieben worden, wie Wasserstoffperoxid (W.F. Armstrong et al., Circulation 66: Suppl. II (1982), II-256), mit Kohlendioxid angereicherte Kochsalzlösung (A.N. De Maria et al., Circulation 60: Suppl. II (1980), II-143), ein Gemisch aus Renografin® und Kochsalz (G. Maurer et al., J. Am. Coll. Cardiol. I(II) (1983), 645), gelatineverkapselte Mikrobläschen (W.F. Armstrong et al., Circulation 66 (1982), 166), in unterschiedlicher Weise stabilisierte Gasbläschen (J. Folkert et al., JACC, Vol. 3, No. 5 (1984), 1219-1226) und durch Mikropartikel stabilisierte Gasbläschen (DE-OS 33 24 745 und EP-OS 0 052 575).

Wenngleich diese herkömmlichen Kontrastmittel Gasbläschen mit einer Teilchengröße von etwa 10 µm oder geringfügig darunter ergeben (siehe die EP-OS 0 052 575 und J. Folkert et al., loc. cit.), vermögen diese herkömmlichen Kontrastmittel namentlich im Hinblick auf die Teilchengröße und die Stabilität der Gasbläschen nicht zu befriedigen, so daß es bislang nicht möglich geworden ist, mit Hilfe solcher Kontrastmittel eine echokardiographische Darstellung des linken Ventrikels durch periphere venöse Kontrastmittelinjektion zu erreichen. In der EP-OS 0123235 und der WO-OS 84/02838 werden Ultraschallkontrast mittel beschrieben, die feste Mikropartikel enthalten. Die Aufgabe der vorliegenden Erfindung besteht somit darin, ein geeignetes festkörperfreies Kontrastmittel für Ultraschalluntersuchungen und insbesondere für die Echokardiographie zu schaffen, welches nicht toxisch ist, Mikrobläschen von Luft oder anderen Gasen in ausreichender Konzentration und Stabilität enthält, und zwar mit einer Teilchengröße von kleiner als 8 µm, so daß diese Mikrobläschen in der Lage sind, die Lungenkapillaren zu passieren, wodurch die Diagnose von Linksherzerkrankungen ohne die Anwendung eines Katheters durch Ultraschalluntersuchung möglich wird.

Diese Aufgabe wird nun gelöst durch die kennzeichnenden Merkmale des Kontrastmittels gemäß Hauptanspruch. Die Unteransprüche betreffen besonders bevorzugte Ausführungsformen dieses Erfindungsgegenstandes sowie ein Verfahren zur Herstellung des beanspruchten Kontrastmittels.

Gegenstand der Erfindung ist daher ein Kontrastmittel für Ultraschalluntersuchungen und insbesondere für die Echokardiographie auf der Grundlage einer Suspension von Gasbläschen in einer pharmakologisch annehmbaren, wäßrigen Trägerflüssigkeit mit einem physiologischen Elektrolytgehalt, das dadurch gekennzeichnet ist, daß die Trägerflüssigkeit

A) ein pharmakologisch annehmbares, d. h. nicht-toxisches und nicht-antigenes Polypeptid und/oder Polysaccharid und/oder ein Derivat davon,
B) ein pharmakologisch annehmbares pflanzliches Öl und
C) ein pharmakologisch annehmbares lösliches Eisen(III)-salz enthält.

Dieses Kontrastmittel ermöglicht den Einschluß von Luft in Form von Mikrobläschen, die bei manueller Suspension der Luft zu 100 % kleiner als 6,4 µm und im bewegten System zu 100 % kleiner als 3,9 µm sind. Dabei zeichnet sich das Kontrastmittel durch seine niedrige Osmolalität und seine gute Verträglichkeit aus. Durch diese Kleinheit der Mikrobläschen und die gute Verträglichkeit ermöglicht dieses Kontrastmittel es erstmals, daß die Mikrobläschen die Lungenkapillaren in ausreichender Menge passieren können und sich damit im linken Vorhof, im linken Ventrikel und in der Aorta ascendens nachweisen lassen können. Dies konnte anhand von 27 Patienten nachgewiesen werden, worauf weiter unten noch eingegangen werden wird. Da die Mikrobläschen im Blut suspendiert werden, läßt sich der Blutfluß darstellen, so daß es ohne weiteres gelingt, unblutig pathologische Zustände in der rechten und in der linken Herzhälfte zu erkennen. Dadurch wird es mit Hilfe des erfindungsgemäßen Kontrastmittels möglich, auf praktisch nicht-invasivem Wege lediglich durch periphere intravenöse Injektion Aussagen über die Flußeigenschaften in den linken Herzabschnitten, über Regurgitationen an der Mitral- und Aortenklappe, über Links-Rechts-Shunts, über arterielle Ultrasonographie und auch über die Myokardperfusion zu machen.

Durch die Kleinheit der in dem erfindungsgemäßen Kontrastmittel enthaltenen Gasbläschen entfällt jegliche Emboliegefahr, da die roten Blutkörperchen im Durchschnitt größer sind als die Gasbläschen, nämlich 6 μm im Durchmesser.

Das erfindungsgemäße Kontrastmittel enthält als Bestandteil A, nämlich als pharmakologisch annehmbares Polypeptid und/oder Polysaccharid oder Derivat davon vorzugsweise Gelatine, Gelatinederivate, wie Oxypolygelatine, abgebaute Gelatine, Gelatine-polysuccinat, vernetzte Polypeptide, Dextrane, vernetzte Dextrane oder Mischungen davon.

Als zweiten Bestandteil enthält das erfindungsgemäße Kontrastmittel ein pharmakologisch annehmbares pflanzliches Öl, welches dazu dient, die Luftbläschen in der wäßrigen Trägerflüssigkeit, die den Bestandteil A enthält, zu stabilisieren. Als pharmakologisch annehmbares pflanzliches Öl kann man erfindungsgemäß vorzugsweise Sojaöl, Maisöl, Sesamöl, Erdnußöl oder Mischungen davon einsetzen. Mit besonderem Vorteil verwendet man fraktioniertes Sojaöl von Arzneimittelqualität.

Als Bestandteil C enthält das erfindungsgemäße Kontrastmittel ein pharmakologisch annehmbares lösliches Eisen-(III)-salz, welches ebenfalls zur Stabilisierung der Gasbläschen und zur Verminderung der Oberflächenspannung des Kontrastmittels dient. Man kann beliebige Lösungen, die dreiwertige Eisenionen enthalten, einsetzen, wobei man jedoch besonders bevorzugt einen Natrium-eisen(III)-gluconat-Komplex verwendet.

Als fakultative Bestandteile kann das beanspruchte Kontrastmittel in der Trägerflüssigkeit einen Zuckeralkohol, wie Sorbit, Maltit, Galactit, Xylit etc., in einer Menge von 0 bis 20 %, bezogen auf den Bestandteil A, enthalten.

Weiterhin ist es von Vorteil, der Trägerflüssigkeit als weiteren Bestandteile E) ein Phospholipid, wie Lecithin, zuzusetzen, welches als oberflächenaktives Mittel dient und über die bessere Suspendierung des pflanzlichen Öls in der wäßrigen Grundflüssigkeit die Mikrogasbläschen stabilisiert.

Die Trägerflüssigkeit des erfindungsgemäßen Kontrastmittels kann als weiteren Bestandteil F) Glycerin oder als Bestandteil G) ein Konservierungsmittel, wie Benzylalkohol oder dergleichen, enthalten.

Das erfindungsgemäße Kontrastmittel enthält einen Elektrolyten in physiologischer Konzentration, beispielsweise 0,9 % Natriumchlorid, wenngleich man als wäßrige Grundflüssigkeit auch eine Ringer-Lösung oder eine Tyrode-Lösung oder auch eine wäßrige Lösung von Maltose, Dextrose, Lactose oder Galactose einsetzen kann:

Die wäßrige Trägerflüssigkeit mit physiologischem Elektrolytgehalt des erfindungsgemäßen Kontrastmittels enthält vorzugsweise 1 bis 5 Gew.-%, bevorzugter 2 bis 4 Gew.-% und insbesondere 3 bis 4 Gew.-% des Bestandteils A, d. h. des pharmakologisch annehmbaren Polypeptids und/oder Polysaccharids und/oder des Derivats davon, 1 bis 10 Gew.-%, bevorzugter 5 bis 8 Gew.-% und insbesondere 6 bis 7,5 Gew.-% des Bestandteils B, d. h. des pharmakologisch annehmbaren pflanzlichen Öls und 0,01 bis 0,05 Gew.-%, vorzugsweise 0,02 bis 0,04 Gew.-% des Bestandteils C, d. h. des pharmakologisch annehmbaren löslichen Eisen(III)-salzes.

Als weitere fakultative Bestandteile kann die Trägerflüssigkeit des beanspruchten Kontrastmittels
0 bis 1 Gew.-% des Bestandteils D, d. h. des Zuckeralkohols,
0 bis 5 Gew.-%, bevorzugter 0,01 bis 2 Gew.-% und noch bevorzugter 0,3 bis 0,5 Gew.-% des Bestandteils E, d. h. des Phospholipids, wie Lecithin, und
0 bis 2 Gew.-%, bevorzugter 0,6 bis 0,9 Gew.-% des Bestandteils F, d. h. Glycerin, enthalten.

In dem erfindungsgemäßen Kontrastmittel ist ein Gas, wie Luft, Stickstoff oder auch ein Edelgas, Helium oder Mischungen daraus, in einer Menge von 1 bis 10 Vol.-% und noch bevorzugter in einer Menge von 3 bis 6 Vol.-% dispergiert, und zwar in Form von Bläschen mit einem Durchmesser von weniger als 7 μm (wobei diese Teilchengröße nach der manuellen Suspension der Gasbläschen in der wäßrigen Trägerflüssigkeit gemessen worden ist). Diese manuelle Suspension besteht darin, die Flüssigkeit in der Gasatmosphäre über einen Drei-Wegehahn 25-mal hin- und herzuspritzen. Natürlich ist es auch möglich, das Gas unter Einwirkung von Ultraschall in der wäßrigen Trägerflüssigkeit zu dispergieren oder hierzu eine beispielsweise elektrisch angetriebene Pumpe einzusetzen.

Durch das Einmischen des Gases in die wäßrige Trägerflüssigkeit entsteht eine homogene Suspension, die etwa 5 bis 10 Minuten stabil ist, wobei beim Stehenlassen bereits nach 2 Minuten eine Phasentrennung erfolgt, indem sich die Bestandteile A und B in verschiedenen Schichten absetzen. Diese Phasentrennung ist bedeutungslos, da die Mikrobläschen in beiden Phasen homogen verteilt sind. Mit zunehmender Dauer kommt es beim Stehenlassen als Folge der Oberflächenspannung an den Bläschen und durch kohäsive Kräfte zu einem Aneinanderreihen der Bläschen im Sinne einer Kette. Auch in diesem Stadium ist eine Passage der Gasbläschen durch die Kapillaren noch möglich. Bei weiterem Stehenlassen erfolgt ein Zusammenfließen von mehreren kleinen Bläschen zu größeren Bläschen, die zuerst an den Grenzflächen, beispielsweise an den Wandungen der Gefäße, auftritt.

Durch die Zugabe der Bestandteile B und C zu der Trägerflüssigkeit des erfindungsgemäßen Kontrastmittels wird diese Stabilisierung der Gasbläschensuspension erreicht. Es ist zwar möglich, die Gasbläschen auch in dem Bestandteil A allein zu suspendieren, wobei jedoch dann eine sehr instabile Suspension erhalten wird, die nach dem Stehenlassen während 1 bis 2 Minuten bereits größere Luftbläschen ergibt, die sich nach 10 Minuten zu einer einzigen Luftblase vereinigt haben.

Ein weiterer Gegenstand der Erfindung ist ein Verfahren zur Herstellung des oben beschriebenen Kontrastmittels, welches darin besteht, daß man ein Gas, wie vorzugsweise Luft, manuell, mechanisch, beispielsweise mit Hilfe einer elektrisch angetriebenen Pumpe, oder unter Einwirkung von Ultraschall in der Trägerflüssigkeit suspendiert.

Bei der Herstellung des erfindungsgemäßen Kontrastmittels kann man als Bestandteil A handelsübliche Produkte einsetzen, die Oxypolygelatine (Gelifundol®), abgebaute Gelatine (Gelafundin® oder Physiogel®), Gelatine-polysuccinat (Thomaegelin® 4 % in Ringeracetat), vernetzte Polypeptide (Haemaccel® 35) und Dextran (Dextranlösung 40 salvia mit 20 % Sorbit®, Infusionslösung 10 % Dexran 40, 20 % Sorbit®, Longasteril 40 mit 20 % Sorbit®, Onkovertin N mit Sorbit®, Rheofusin S 20®, Rheomacrodex mit 10 % Sorbit 20 %®, Dextranlösung 40 elektrolytfrei®, Dextranlösung 40 salvia mit 0,9 % NaCl®, Infusionslösung 10 % Dextran 40, 0,9 % NaCl®, Longasteril 40 kochsalzfrei®, Onkovertin N®, Onkovertin N kochsalzfrei®, Parenteral D 40®, Plasmafusin 40 E®, Rheofusin®, Rheofusin E®, Rheofusin kochsalzfrei®, Rheomacrodex 10 %®, Rheomacrodex 10 % natriumchloridfrei G®, Rheomacrodex 10 % natriumchloridfrei S®, Thomaedex 40 kochsalzfrei mit 5 % Sorbit®, Thomaedex 40 mit NaCl®, Dextran-Lösung 60®, Dextran-Lösung 75 salvia mit 0,9 % NaCl®, Infusionslösung 6 % Dextran 70 0,9 % Natriumchlorid®, Longasteril 70 mit Elektrolyten®, Macrodex 4,5 % RL®, Macrodex 6 %®, Onkovertin 6 %®, Plasmafusin 60®, Thomaedex 60 mit NaCl®) enthalten.

Als Bestandteil B kann man ebenfalls ein Handelsprodukt einsetzen, nämlich Intralipid® 20 % Vitrum, Intralipid® 10 % Vitrum, Lipofundin® MCT 10 %, Lipofundin® S 10 % oder 20 %, Nutrifundin® und Tutolipid®.

Auch als Bestandteil C kann man Handelsprodukte verwenden, wie Ferrlecit®, Ferreophor®, Ferrum Hausmann® oder Jectofer®.

Bei der Anwendung des erfindungsgemäßen Kontrastmittels wird dieses nach dem Suspendieren des Gases in der Trägerflüssigkeit intravenös injiziert, wonach mit 10 ml physiologischer (0,9 %-iger) Kochsalzlösung nachgespritzt wird, um erstens eine Bolusinjektion zu erreichen und zweitens das unkontrollierte nachfolgende Anfluten des Kontrastmittels aus den gefüllten Venen zu verhindern. In dieser Weise lassen sich kontrollierte Bedingungen erreichen, die beispielsweise auch eine videosensitometrische Auswertung beispielsweise des rechten Ventrikels ermöglichen.

Aufgrund der Tatsache, daß das erfindungsgemäße Kontrastmittel eine sehr stabile Dispersion von kleinen Mikrobläschen mit einer Teilchengröße unterhalb der Teilchengröße der roten Blutkörperchen ergibt, wird die Passage des Kontrastmittels durch die Lungenkapillaren und andere feinste Gefäße möglich, was zur Folge hat, daß das Kontrastmittel über die Armvene injiziert werden kann und über die Lungenkapillaren in ausreichender Menge in den linken Herzvorhof, den linken Ventrikel und die Aorta ascendens überführt wird und sich dort nachweisen läßt. Da die Mikrobläschen im Blut schwimmen, läßt sich der Blutfluß darstellen, wodurch praktisch unblutig pathologische Zustände in der rechten und der linken Herzhälfte erkannt werden können. Darüber hinaus ermöglichen die feinen Gasbläschen des erfindungsgemäßen Kontrastmittels nicht nur die Untersuchung des Herzens durch Echokardiographie, sondern auch die Untersuchung anderer Organe, wie der Leber, durch Ultraschallechos. Hierzu sind die herkömmlichen Kontrastmittel aufgrund ihrer physiologischen Bestandteile und ihrer Bläschengröße nicht geeignet, da die feinen Gefäße dieser Organe nicht mehr durchströmt werden.

Die geringe Perfusionszeit des erfindungsgemäßen Kontrastmittels, die in der Größenordnung derjenigen des Bluts liegt, hat zur Folge, daß die Strömungsverhältnisse des Bluts durch das Kontrastmittel nicht beeinträchtigt werden, was wesentlich genauere und bessere diagnostische Möglichkeiten eröffnet.

Die folgenden Beispiele dienen der weiteren Erläuterung der Erfindung.

**Beispiel 1**

Man bereitet ein Kontrastmittel aus den folgenden Bestandteilen Al, Bl, Cl und Luft in den nachfolgend angegebenen Mengenverhältnissen, wobei die Bestandteile Al, Bl und Cl die erfindungsgemäßen Bestandteile A, B und C enthalten und die folgende Zusammensetzung besitzen:

Bestandteil Al (Gelifundol®):

55,0 g Oxypolygelatine

5,84 g Natriumchlorid

2,52 g Natriumhydrogencarbonat

0,19 g Ethylendiamintetraessigsäure (Dinatriumsalz)

0,07 g Calciumchlorid

ad 1000 ml Wasser für Injektionszwecke

Bestandteil Bl (Intralipid® 20 % Vitrum):

200 g fraktioniertes Sojaöl

12 g Eigelb-lecithin

22,5 g Glycerin

ad 1000 ml Wasser für Injektionszwecke

Bestandteil Cl (Ferrlecit®):

62,5 mg Eisen (als Natrium-eisen(III)-gluconat-Komplex)

45 mg Benzylalkohol (zur Konservierung)

ad 5 ml Wasser für Injektionszwecke.

Unter Anwendung der obigen Bestandteile Al, Bl, Cl und Luft stellt man die folgenden Dosierungen des erfindungsgemäßen Kontrastmittels her:

Dosis 1:

5 ml Bestandteil Al

3 ml Bestandteil Bl

0,2 ml Bestandteil Cl

0,5 ml Luft

= 8,7 ml

Dosis 2:

10 ml Bestandteil Al

6 ml Bestandteil Bl

0,4 ml Bestandteil Cl

1 ml Luft

= 17,4 ml

Dosis 3:

18 ml Bestandteil Al

8 ml Bestandteil Bl

0,5 ml Bestandteil Cl

1,5 ml Luft

= 28 ml

Die oben angegebenen Dosierungen wurden auf zwei Injektionsspritzen verteilt und über einen Drei-Wegehahn mindestens 25-mal schnell hin- und hergespritzt, wodurch eine Mischung entsteht, die keinerlei sichtbare Luftbläschen enthält.

Die Osmolalität dieser Mischungen beträgt 345 mosmol/kg.

Zur Orientierung wurden die erhaltenen Mischungen zunächst lichtmikroskopisch untersucht, wobei sich gezeigt hat, daß die erhaltenen Luftbläschen zu über 99 % eine Teilchengröße von weniger als 5 $\mu$m aufweisen.

Wegen der Deformierungsmöglichkeit der kleinen Luftbläschen durch das Deckgläschen sowie die adhäsiven Kräfte zwischen Objektträger-Lösung und Deckgläschen-Lösung wurde die Größenbestimmung mit Hilfe eines Lasergeräts wiederholt, wobei die Größenbestimmung der Mikrobläschen sowohl in einer Standzelle als auch im bewegten System erfolgte. Die Lösung wurde dabei im bewegten System einmal manuell, im anderen Fall mit Ultraschall suspendiert. Die hierbei erhaltenen Ergebnisse sind in der nachfolgenden

Tabelle I zusammengestellt:

## TABELLE I

| Standzelle | | Bewegtes System | | Bewegtes System | |
|---|---|---|---|---|---|
| Manuelle Suspension | | Manuelle Suspension | | Mit Ultraschall suspendiert | |
| kleiner als ($\mu$m) | % der Bläschen | kleiner als ($\mu$m) | % der Bläschen | kleiner als ($\mu$m) | % der Bläschen |
| 6,4 | 100 | 3,9 | 100 | 5,0 | 100 |
| 5,0 | 99,7 | 3,0 | 99,8 | 3,9 | 99,9 |
| 3,9 | 95,0 | 2,4 | 98,2 | 3,0 | 96,9 |
| 3,0 | 62,3 | 1,0 | 91,8 | 2,4 | 88,4 |
| 2,4 | 24,6 | 1,5 | 77,5 | 1,9 | 77,1 |
| 1,9 | 14,1 | 1,2 | 66,7 | 1,5 | 59,6 |
| 1,5 | 11,0 | | | 1,2 | 42,1 |
| 1,2 | 10,4 | | | | |

Wie aus der obigen Tabelle I hervorgeht, erhält man eine Suspension mit Gasbläschen mit einer Teilchengröße unterhalb der Teilchengröße der roten Blutkörperchen, wobei diese homogene Suspension 5 bis 10 Minuten stabil ist und die dann auftretende Phasentrennung unproblematisch ist, da die Mikrobläschen in beiden Phasen homogen verteilt sind.

## Beispiel 2

Das erfindungsgemäße Kontrastmittel wurde in den obigen Dosierungen 1 bis 3 intravenös über die rechte oder linke Vena cubitalis an 27 Patienten im Alter von 17 bis 65 Jahren ($\bar{x}$ = 39 ± 19) verabreicht, um diagnostische Aussagen über die Tricuspidal-, Mitral- und Aortenklappe und über das linksventrikuläre Flußverhalten treffen zu können. Die einzige Voraussetzung für die echokardiographische Untersuchung war eine gute Schallbarkeit der Patienten. Zu Beginn wurde immer die Dosis 1 verwendet. Da die Injektionen von allen Patienten ohne die geringsten Nebenwirkungen vertragen wurden, erfolgte bei entsprechender diagnostischer Fragestellung eine Dosiserhöhung entsprechend den Dosierungen 2 und 3. In der nachfolgenden Tabelle II wird eine Übersicht über das Spektrum der Erkrankungen der untersuchten Patienten gegeben.

TABELLE II

| Nr. | Pat. | Alter (Jahren) | Diagnosen | EKG-Veränderungen unter Injektion |
|---|---|---|---|---|
| 1 | H.P. | 57 | Z.n.Vorderwandinfarkt | keine |
| 2 | T.B. | 18 | Thrombus re.Vorhof | " |
| 3 | G.H. | 25 | Thrombus li.Ventrikel | " |
| 4 | P.M. | 20 | AKE (Björk Shiley V.) | " |
| 5 | N.G. | 29 | TI Grad II | " |
| 6 | A.D. | 73 | AS Grad IV | " |
| 7 | S.K. | 31 | AS Grad II,AI Grad III | " |
| 8 | R.R. | 57 | CC Stadium III | " |
| 9 | M.E. | 45 | Rhythmusstörungen | " |
| 10 | A.S. | 60 | Lungenemphysem | " |
| 11 | V.R. | 49 | MKE (Wada-Cutter V.) | " |
| 12 | J.A. | 17 | bicuspide Aortenklappe | " |
| 13 | H.H. | 47 | Z.n.Lungenembolien,TI | " |
| 14 | N.B. | 51 | AI Grad II | " |
| 15 | K.K. | 18 | Mitralklappenprolaps | " |
| 16 | A.W. | 19 | PS Grad I,PI Grad I | " |
| 17 | P.G. | 59 | fehlmündende Lungenv. | " |
| 18 | C.E. | 60 | MKE (Björk Shiley V.) | " |
| 19 | S.W. | 19 | TI Grad II - III | " |
| 20 | P.P. | 16 | AS Grad I - II,Vd. MI | " |
| 21 | H.W. | 48 | AI Grad IV | " |
| 22 | H.P. | 55 | AI Grad IV | " |
| 23 | A.V. | 45 | Linksschenkelblock | " |
| 24 | M.D. | 49 | CC Stadium II - III | " |
| 25 | S.L. | 26 | pulmonale Hypertonie | " |
| 26 | G.H. | 20 | IHSS | " |
| 27 | F.H. | 65 | MKE (Björk Shiley V.) | " |

Abkürzungen : Z.n. = Zustand nach , AKE = Aortenklappenersatz, V.= Ventil, TI = Tricuspidalinsuffizienz, AS = Aortenstenose,AI = Aorteninsuffizienz, CC = congestive Cardiomyopathie,MKE = Mitralklappenersatz,PS = Pulmonalstenose, PI = Pulmonalinsuffizienz, IHSS = idiopathische hypertrophe Subaortenstenose

Bei dieser Injektion des erfindungsgemäßen Kontrastmittel kam es in allen angewandten Dosierungen weder bei der Herzfrequenz noch beim systolischen oder diastolischen Blutdruck zu signifikanten Veränderungen, wozu auf die nachfolgende Tabelle III verwiesen werden darf.

TABELLE III

| | Herzfrequenz | | | RR syst. | | | RR diast. | | |
|---|---|---|---|---|---|---|---|---|---|
| | vor | nach | S | vor | nach | S | vor | nach | S |
| Dosis 1 | 75±18 | 73±18 | p>0,05 | 121±13 | 121±14 | p>0,05 | 77±17 | 77±17 | p>0,05 |
| Dosis 2 | 71±14 | 71±13 | p>0,05 | 121±14 | 124±13 | p>0,05 | 77±17 | 77±17 | p>0,05 |
| Dosis 3 | 71±19 | 72±18 | p>0,05 | 121±10 | 127±16 | p>0,05 | 72±22 | 73±23 | p>0,05 |

Mit zunehmender Menge des intravenös verabreichten Kontrastmittels kommt es zu einem frühzeitigeren Nachweis des Kontrastmittels im linken Ventrikel, ohne daß die Signifikanzgrenze bei der Dosis 1 gegen die Dosis 2 bzw. Dosis 3 erreicht wird. Außerdem kommt es mit der Dosiserhöhung zu einer verlängerten Nachweisbarkeit des Kontrastmittels im linken Ventrikel, wobei sich signifikante Veränderungen nachweisen lassen, wozu auf die nachfolgende Tabelle IV verwiesen sei.

TABELLE IV

| Dauer des Nachweises der Bläschen im li.Ve. (s) | Anzahl der Systolen bis zum Nachweis im li.Ve. |
|---|---|
| Dosis 1 (n=23)  22 ± 15 | 4,5 ± 3,4 |
| Dosis 2 (n=19)  39 ± 34 ☆ | 3,6 ± 2 |
| Dosis 3 (n=5)  75 ± 40 ☆☆ | 2,8 ± 2,1 |

☆ $p < 0,05$
☆☆ $p < 0,01$

Insgesamt konnten bei neun Patienten linksventrikuläre Funktionsstörungen nachgewiesen werden. Bei vier Patienten konnte eine Aorteninsuffizienz diagnostiziert werden (Patient Nr. 2, 4, 21 und 22), wobei dies angiokardiographisch bestätigt werden konnte. Bei einer Patientin (Nr. 18) konnte eine Regurgitation über eine Björk-Shiley-Prothese in Mitralposition eindeutig nachgewiesen werden, wobei bei dieser Patientin das Doppler-Echokardiogramm keine Regurgitation nachweisen konnte. Bei einem Patienten konnte ein Leck eines Björk-Shiley-Ventils ausgeschlossen werden, wobei eine Übereinstimmung mit dem Doppler-Echokardiogramm bestand. Des weiteren konnte bei drei Patienten eine klinisch vermutete Mitralinsuffizienz ausgeschlossen werden (Nr. 7, 20, 24). Außerdem wurde bei einem Patienten (Nr. 12) eine Myokardanfärbung gesehen.

Es ist also festzuhalten, daß es mit Hilfe des erfindungsgemäßen Kontrastmittels gelingt, durch periphere venöse Injektion über den rechten Vorhof und den rechten Ventrikel die Lungenkapillaren zu passieren und damit den linken Ventrikel und die Aorta ascendens mit dem Kontrastmittel zu erreichen.

Bei keinem der untersuchten Patienten traten irgendwelche Nebenwirkungen auf, auch nach der Passage in den linken Ventrikel und anschließend in das zerebrale Gefäßsystem konnten keine subjektiven oder objektiven Erscheinungen festgestellt werden. Auch bei zwei Patienten mit bekannter Iodallergie (bei einem Patienten mußte die Herzkatheteruntersuchung unterbrochen werden) und bei einem Patienten mit einer pflasterallergie traten keine Hebenwirkungen auf. Die Herzfrequenz änderte sich vor und nach der Injektion nicht. Der Blutdruck zeigte ebenfalls keine statistisch signifikanten Veränderungen. Bei der Dosis 2 kam es zu einer Erhöhung des systolischen Blutdrucks um 3 mmHg und bei der Dosis 3 um 6 mmHg (p > 0,05).

Mit zunehmender Dosierung kommt es zu einem frühzeitigeren Nachweis der Mikrobläschen im linken Ventrikel (Tabelle IV), wobei die Unterschiede sich bei den vorhandenen Patientenzahlen noch nicht statistisch sichern ließen. In der Dosis 1 werden im Mittel 4,5 rechtsventrikuläre Systolen benötigt, in der Dosis 3 2,8 rechtsventrikuläre Systolen. Dies weist darauf hin, daß die Erscheinungszeit abhängig ist von der Anzahl der Mikrobläschen und der echokardiographischen Nachweisbarkeit, d. h. von dem aufzulösenden System. Die Anzahl von 2,8 Systolen weist aber auch darauf hin, daß sich die Nachweisbarkeit schon fast im Rahmen der physiologischen Passierbarkeit durch die Lungenkapillaren befindet. Auch die Dauer des Nachweises im linken Ventrikel ist dosisabhängig (Tabelle IV), d. h. die Dauer des Nachweises ist ebenfalls von der Anzahl der injizierten Mikrobläschen abhängig. Außerdem weist die lange Nachweisbarkeit der Mikrobläschen in der Dosis 3 (75 ± 40 s) auf eine gute Stabilität der Mikrobläschen und damit des beanspruchten Kontrastmittels hin.

Mit Hilfe des erfindungsgemäßen Kontrastmittels, welches die Lungenkapillaren passiert, ist es möglich, die Kontrastmittel-Echokardiographie aus ihren bisherigen Indikationsgebieten herauszuführen und die unblutige Flußbeurteilung an den linksventrikulären Klappen und im linken Ventrikel möglich zu machen. Über die Doppler-Echokardiographie hinaus ist ein Nachweis des Flusses in vielen Ebenen, eine Beurteilung auch von Kunstklappen im linken Ventrikel sowie eine visuelle dynamische Darstellung des Blutflusses in allen Strukturen des linken Ventrikels möglich. So kann unter anderem auch das Aufprallen der Mikrobläschen auf das Klappengerüst bzw. auf den Klappendeckel beobachtet werden. Darüber hinaus ermöglicht das erfindungsgemäße Kontrastmittel die Beurteilung von intrapulmonalen Rechts-Links-Shunts sowie der Passagezeiten in den Lungenkapillaren.

## Patentansprüche

1. Kontrastmittel für Ultraschalluntersuchungen auf der Grundlage einer Suspension von Gasbläschen in einer physiologisch annehmbaren, wäßrigen Trägerflüssigkeit mit einem physiologischen Elektrolytgehalt, **dadurch gekennzeichnet**, daß die Trägerflüssigkeit

   A) ein pharmakologisch annehmbares Polypeptid und/oder Polysaccharid und/oder ein Derivat davon,

   B) ein pharmakologisch annehmbares pflanzliches Öl und

   C) ein pharmakologisch annehmbares lösliches Eisen(III)-salz

   enthält.

2. Kontrastmittel nach Anspruch 1, **dadurch gekennzeichnet**, daß die Trägerflüssigkeit als weiteren Bestandteil

   D) 0 bis 20 % (bezogen auf den Bestandteil A) eines Zuckeralkohols enthält.

3. Kontrastmittel nach den Ansprüchen 1 und 2, **dadurch gekennzeichnet**, daß die Trägerflüssigkeit als weiteren Bestandteil

   E) ein Phospholipid enthält.

4. Kontrastmittel nach den Ansprüchen 1 bis 3, **dadurch gekennzeichnet,** daß die Trägerflüssigkeit als weiteren Bestandteil

   F) Glycerin enthält.

5. Kontrastmittel nach mindestens einem der vorhergehenden Ansprüche, **dadurch gekennzeichnet**, daß die Trägerflüssigkeit als weiteren Bestandteil

   G) ein Konservierungsmittel enthält.

6. Kontrastmittel nach den Ansprüchen 1 bis 5, **dadurch gekennzeichnet,** daß es als Bestandteil A) Oxypolygelatine, abgebaute Gelatine, Gelatine-polysuccinat, ein vernetztes Polypeptid, ein Dextran oder Mischungen davon enthält.

7. Kontrastmittel nach einem der vorhergehenden Ansprüche, **dadurch gekennzeichnet**, daß die Trägerflüssigkeit als pharmakologisch annehmbares pflanzliches Öl Sojaöl, Maisöl, Sesamöl, Erdnußöl oder Mischungen davon enthält.

**8.** Kontrastmittel nach einem der vorhergehenden Ansprüche, **dadurch gekennzeichnet**, daß die Trägerflüssigkeit als pharmakologisch annehmbares lösliches Eisen(III)-salz einen Natrium-eisen(III)-gluconat-Komplex enthält.

**9.** Kontrastmittel nach einem der vorhergehenden Ansprüche, **dadurch gekennzeichnet**, daß die Trägerflüssigkeit als Zuckeralkohol Sorbit, Maltit, Galactit und/oder Xylit enthält.

**10.** Kontrastmittel nach einem der vorhergehenden Ansprüche, **dadurch gekennzeichnet**, daß die Trägerflüssigkeit als Phospholipid Lecithin enthält.

**11.** Kontrastmittel nach den Ansprüchen 1 bis 10, **dadurch gekennzeichnet**, daß die Trägerflüssigkeit
1 bis 5 Gew.-% des Bestandteils A),
1 bis 10 Gew.-% des Bestandteils B),
0,01 bis 0,05 Gew.-% des Bestandteils C),
0 bis 1 Gew.-% des Bestandteils D),
0 bis 5 Gew.-% des Bestandteils E),
0 bis 2 Gew.-% des Bestandteils F) und
1 bis 10 Vol.-% eines Gases in Form von Bläschen mit einem Durchmesser von weniger als 7 $\mu$m enthält.

**12.** Verfahren zur Herstellung des Kontrastmittels gemäß mindestens einem der vorhergehenden Ansprüche, **dadurch gekennzeichnet**, daß man ein Gas manuell, mechanisch oder unter Einwirkung von Ultraschall in der Trägerflüssigkeit suspendiert.

## Claims

**1.** Contrast medium for ultrasonic examinations based on a suspension of gas bubbles in a physiologically acceptable aqueous liquid vehicle having a physiological electrolyte content, characterized in that the liquid vehicle contains
A) a pharmacologically acceptable polypeptide and/or polysaccharide and/or a derivative thereof,
B) a pharmacologically acceptable vegetable oil and
C) a pharmacologically acceptable soluble iron(III) salt.

**2.** Contrast medium according to Claim 1, characterized in that the liquid vehicle contains as further constituent
D) 0 to 20% (based on constituent A) of a sugar alcohol.

**3.** Contrast medium according to Claims 1 and 2, characterized in that the liquid vehicle contains as further constituent
E) a phosphololipid.

**4.** Contrast medium according to Claims 1 to 3, characterized in that the liquid vehicle contains as further constituent
F) glycerol.

**5.** Contrast medium according to at least one of the preceding claims, characterized in that the liquid vehicle contains as further constituent
G) a preservative.

**6.** Contrast medium according to Claims 1 to 4, characterized in that it contains as constituent A) oxypolygelatin, degraded gelatin, gelatin polysuccinate, a cross-linked polypeptide, a dextran or mixtures thereof.

**7.** Contrast medium according to one of the preceding claims, characterized in that the liquid vehicle contains as pharmacologically acceptable vegetable oil soya oil, maize oil, sesame oil, arachis oil or mixtures thereof.

**8.** Contrast medium according to one of the preceding claims, characterized in that the liquid vehicle

EP 0 212 568 B1

contains as pharmacologically acceptable soluble iron(III) salt a Sodium iron( III) gluconate complex.

9. Contrast medium according to one of the preceding claims, characterized in that the liquid vehicle contains as sugar alcohol sorbitol, maltitol, galactitol and/or xylitol.

10. Contrast medium according to one of the preceding claims, characterized in that the liquid vehicle contains as phospholipid lecithin.

11. Contrast medium according to Claims 1 to 10, characterized in that the liquid vehicle contains
1 to 5% by weight of constituent A),
1 to 10% by weight of constituent B),
0.01 to 0.05% by weight of constituent C),
0 to 1% by weight of constituent D),
0 to 5% by weight of constituent E),
0 to 2% by weight of constituent F) and
1 to 10% by volume of a gas in the form of bubbles with a diameter of less than 7 $\mu$m.

12. Process for the preparation of the contrast medium according to at least one of the preceding claims, characterized in that a gas is suspended manually, mechanically or by the action of ultrasound in the liquid vehicle.

**Revendications**

1. Produit de contraste pour analyses par ultrasons, à base d'une suspension de petites bulles de gaz dans un véhicule liquide physiologiquement acceptable et aqueux, contenant une quantité d'électrolyte physiologique, caractérisé en ce que le véhicule liquide contient :
   A) un polypeptide pharmacologiquement acceptable et/ou un polysaccharide et/ou un de ses dérivés,
   B) une huile végétale pharmacologiquement acceptable, et
   C) un sel ferrique soluble pharmacologiquement acceptable.

2. Produit de contraste selon la revendication 1, caractérisé en ce que le véhicule liquide contient comme composant supplémentaire :
   D) 0 à 20 % (par rapport au composant A) d'un alcool de sucre.

3. Produit de contraste selon les revendications 1 et 2, caractérisé en ce que le véhicule liquide contient comme composant supplémentaire : E) un phospholipide.

4. Produit de contraste selon les revendications 1 à 3, caractérisé en ce que le véhicule liquide contient comme composant supplémentaire : F) de la glycérine.

5. Produit de contraste selon au moins l'une des revendications précédentes, caractérisé en ce que le véhicule liquide contient comme composant supplémentaire : G) un agent de conservation.

6. Produit de contraste selon les revendications 1 à 5, caractérisé en ce qu'il contient comme composant A) de l'oxypolygélatine, de la gélatine décomposée, du polysuccinate de gélatine, un polypeptide réticulé, un dextrane ou des mélanges de ces substances.

7. Produit de contraste selon l'une des revendications précédentes, caractérisé en ce que le véhicule liquide contient comme huile végétale pharmacologiquement acceptable, de l'huile de soja, de l'huile de maïs, de l'huile de sésame, de l'huile d'arachide ou des mélanges de ces huiles.

8. Produit de contraste selon l'une des revendications précédentes, caractérisé en ce que le véhicule liquide contient comme sel ferrique soluble pharmacologiquement acceptable, un complexe de sodium-fer(III)-gluconate.

9. Produit de contraste selon l'une des revendications précédentes, caractérisé en ce que le véhicule liquide contient comme alcool de sucre, du sorbitol, de la maltite, de la galactite et/ou de la xylite.

12

**10.** Produit de contraste selon l'une des revendications précédentes, caractérisé en ce que le véhicule liquide contient de la lécithine comme phospholipide.

**11.** Produit de contraste selon les revendications 1 à 10, caractérisé en ce que le véhicule liquide contient :

1 à 5 % en poids du composant A),
1 à 10 % en poids du composant B),
0,01 à 0,05 % en poids du composant C),
0 à 1 % en poids du composant D),
0 à 5 % en poids du composant E),
0 à 2 % en poids du composant F), et
1 à 10 % en volume d'un gaz sous forme de petites bulles d'un diamètre inférieur à 7 $\mu$m.

**12.** Procédé de préparation du produit de contraste selon au moins l'une des revendications précédentes, caractérisé en ce qu'on met un gaz en suspension dans le véhicule liquide, manuellement, mécaniquement ou sous l'action d'ultrasons.